# EUROPÄISCHE PATENTANMELDUNG

(11) **EP 3 170 558 A1**
(43) Veröffentlichungstag der Anmeldung: **24.05.2017**
(21) Anmeldenummer: 15195303.1
(22) Anmeldetag: 19.11.2015
(51) Int. Cl.: B01J 37/02, B01J 37/06, B01J 37/12, B01J 37/14, B01J 21/04, B01J 21/08, B01J 21/10, B01J 23/52, B01J 23/89, B01J 35/00, B01J 35/02, B01J 35/10

(54) **GOLD-BASIERTEN KATALYSATOR FÜR DIE OXIDATIVE VERESTERUNG VON ALDEHYDEN ZU CARBONSÄUREESTERN**

(71) Anmelder: Evonik Röhm GmbH, 64293 Darmstadt (DE)
(72) Erfinder: Die Erfindernennung liegt noch nicht vor

(57) **Zusammenfassung**

Die vorliegende Erfindung betrifft neuartige Katalysatoren für die oxidative Veresterung, mittels derer beispielsweise (Meth)acrolein zu Methyl(meth)acrylat umgesetzt werden kann. Die erfindungsgemäßen Katalysatoren zeichnen sich dabei insbesondere durch eine hohe mechanische und chemische Stabilität auch über sehr lange Zeiträume aus. Dies betrifft insbesondere eine Verbesserung der Katalysatorstandzeit, der Aktivität und der Selektivität gegenüber Katalysatoren des Standes der Technik, die in Medien mit auch geringem Wassergehalt im kontinuierlichen Betrieb relativ schnell Aktivität und/oder Selektivität verlieren.

## Beschreibung

### Gebiet der Erfindung

Die vorliegende Erfindung betrifft neuartige Katalysatoren für die oxidative Veresterung, mittels derer beispielsweise (Meth)acrolein zu Methyl(meth)acrylat umgesetzt werden kann. Die erfindungsgemäßen Katalysatoren zeichnen sich dabei insbesondere durch eine hohe mechanische und chemische Stabilität sowie durch eine gute katalytische Performance auch über sehr lange Zeiträume aus. Dies betrifft insbesondere eine Verbesserung der Katalysatorstandzeit, der Aktivität und der Selektivität gegenüber Katalysatoren des Standes der Technik, die in Medien mit auch geringem Wassergehalt im kontinuierlichen Betrieb relativ schnell Aktivität und/oder Selektivität verlieren.

### Stand der Technik

Die katalytische oxidative Veresterung von Aldehyden zur Herstellung von Carbonsäureestern ist im Stand der Technik umfänglich beschrieben. So kann man z.B. derart sehr effizient Methylmethacrylat aus Methacrolein (MAL) und Methanol herstellen. Insbesondere in US 5,969,178 und in US 7,012,039 wird ein Verfahren zur kontinuierlichen Herstellung von MMA aus Isobuten oder tert-Butanol beschrieben. Dabei weist das Verfahren die folgenden Schritte auf: 1) Oxidation von Isobuten oder tert-Butanol zu Methacrolein und 2) Direkte oxidative Veresterung von MAL mit Methanol zu MMA mit einem Pd-Pb-Katalysator, der sich auf einem oxidischen Träger befinden.

Jedoch weisen sämtliche aus dem Stand der Technik bekannten Katalysatoren bei längeren Standzeiten einen relevanten Verlust an Selektivität und/oder Aktivität auf. So werden zum Beispiel in EP 1 393 800 zwar gute Aktivitäten und Selektivitäten beschreiben, es wird jedoch gleichzeitig keine Auskunft über die Lebensdauer der Katalysatoren gegeben. Es handelt sich dabei um einige Gold-haltige Katalysatoren, wobei die als aktive Oxidationsspezies beschriebenen katalytischen Goldpartikel insbesondere einem durchschnittlichen Durchmesser von weniger als 6 nm aufweisen. Besagte Goldpartikel befinden sich verteilt auf einem Siliziumoxid- bzw. einem TiO₂/SiO₂-Träger. Als zusätzliche Aktivkomponenten außer Gold, enthalten solche Katalysatoren unter anderem auch andere Metalle. Die Herstellung erfolgt durch Auftragen des Goldsalzes und weiteren Metallsalzen auf einen oxidischen Träger und eine anschließende thermische Behandlung in Gegenwart von Wasserstoff als Reduktionsmittel. Für die Umsetzung von Pyruvaldehyd zu Ethylpyruvat wird dabei z.B. auch ein Gold- und Kobalt-haltiger Katalysator auf einem TiO₂-Träger beschrieben. Dabei liegt Kobalt in diesem Katalysator in metallischer Form {Co(0)} vor. Die Selektivität zum Zielprodukt (Ethylpyruvat) beträgt in diesem Fall 81% bei Raum-Zeit-Ausbeute von 24 mol/kg Kat*h. Die Selektivitäten von anderen Gold-haltigen Katalysatoren (ohne Kobalt) zu MMA werden bei einem Gehalt von 4,5 Gew% Au mit bis zu 93% und die Raum-Zeit-Ausbeute mit bis zu 50,7 mol MMA/kg Kat*h angegeben.

Haruta et al beschreiben in J. Catal. 1993, Vol. 144, pp 175-192, dass Goldnanopartikel aufgetragen auf übergangsmetalloxidische Träger, wie TiO₂, Fe₂O₃ oder Co₃O₄ aktive Oxidationskatalysatoren darstellen. Dabei spielt eine Wechselwirkung zwischen Gold und Übergangsmetall eine entscheidende Rolle für die Katalysatoraktivität.

In der US 6,040,472 werden alternative Katalysatoren, die jedoch im Vergleich nur zu unzureichenden Aktivitäten und Selektivitäten zu MMA führen, beschrieben. Es handelt sich in diesem Fall um Pd-Pbhaltige Katalysatoren mit Schalenstruktur. Die Selektivitäten zu MMA werden mit bis zu 91 % und die Raum-Zeit-Ausbeute mit bis zu 5,3 mol angegeben.

In EP 2 177 267 und EP 2 210 664 werden nickelhaltige Katalysatoren mit Schalenstruktur beschrieben. Selektivität zu MMA beträgt bei diesen Katalysatoren bis zu 97%. Die Raum-Zeit-Ausbeute wird mit 9,7 mol MMA/(kg h) bei einem Goldanteil im Katalysator von ca. 1 Gew% beschrieben. Ein NiOₓ/Au-Katalysator zeigt laut Beispielen deutlich bessere Aktivitäten und Selektivitäten zu MMA, während andere Kombinationen, wie z.B. Au mit CuO oder auch Co₃O₄ viel weniger aktiv und selektiv sind.

Auch beschreibt die EP 2 177 267 in Vergleichsbeispiel 7 eine Herstellung von Au/Co₃O₄ haltigen Katalysatoren, ausgehend von Kobaltnitrat und Goldsäure durch gleichzeitiges Auftragen von Au und Co auf einen SiO₂/MgO-Träger. Diese Methode der Auftragung führt erfahrungsgemäß für NiO/Au-Katalysator zu den besten Ergebnissen. Nicht jedoch bei der Verwendung von Kobalt, da hier mit der Verwendung des resultierenden Katalysators zur Herstellung von MMA aus Methacrolein nur 2,6% Umsatz und 45,8% Selektivität bei einer Raum-Zeit-Ausbeute (RZA) von 0,3 mol/(kg h) erzielt werden. Das Vergleichsbeispiel 6 des gleichen Patents beschreibt die Synthese und Verwendung eines Au/Fe₃O₄-Katalysators für die gleiche Umsetzung. Mit diesem Katalysator werden dann 10,4% Umsatz und 55,2% Selektivität an MMA bei einer RZA von 1,4 mol/(kg h) erreicht.

Die EP 2 210 664 offenbart einen Katalysator, der im Außenbereich, in Form einer so genannten Egg-Shell-Struktur, Nickeloxid und Goldnanopartikel auf einem Träger aus SiO₂, Al₂O₃ und einem basischen Element, insbesondere einem Alkali- oder Erdalkalimetall, aufweisen. Das Nickeloxid ist dabei an der Oberfläche angereichert, jedoch auch in tieferen Schichten des Katalysatorpartikels in geringeren Konzentrationen enthalten. Ein solcher Katalysator zeigt sehr gute Aktivitäten und Selektivitäten. Jedoch ist der Katalysator hergestellt nach der erfindungsgemäßen Herstellungsvorschrift aus dieser Anmeldung relativ abriebsempfindlich und instabil, was ein Vergleichsbeispiel im weiteren Text zeigt. Dadurch hat man nur relativ geringe Standzeiten zur Verfügung.

US 2013/0172599 wiederum beschreibt ein siliziumhaltiges Material bestehend aus Si, Al und einer basischen dritten Komponente sowie einem Metall mit erhöhter Säurebeständigkeit als vierte Komponente. Bei dieser vierten Komponente handelt es sich um Ni, Co, Zn oder Fe, die gleichmäßig in dem Träger verteilt sind. Eine Mischung von Si, Al, dem basischen Element und der vierten Komponente bei der Herstellung dieses Materials sorgt für eine solche gleichmäßige Verteilung dieser vierten Komponente im gesamten Träger. Dieses Material kann als Träger für Edelmetall-haltige Katalysatoren eingesetzt werden. Eine bevorzugte Katalysatorvariante für die oxidative Veresterung von Methacrolein zu MMA beinhaltet einen Au-Katalysator geträgert auf einem SiO₂-Al₂O₃-MgO-NiO Material.

Insgesamt ergibt sich aus dem Stand der Technik das Bild, dass Kombinationen von Goldnanopartikeln mit anderen Übergangsmetallen wie Kobalt, Zink oder Eisen verglichen mit Nickel nur unzureichende Aktivitäten und/oder Selektivitäten als Katalysator in der Synthese von MMA aus Methacrolein zu zeigen scheinen.

### Aufgabe

Aufgabe der vorliegenden Erfindung war primär, die Herstellung eines neuartigen Katalysators für eine hoch selektive oxidative Veresterung von Aldehyden zu Carbonsäureestern zur Verfügung zu stellen. Dabei soll dieser Katalysator eine hohe mechanische und chemische Stabilität, insbesondere in Wasser- und Carbonsäure-haltigen Gemischen, aufweisen und über ein gegenüber dem Stand der Technik insgesamt besseres Gesamtbild aus Aktivität, Selektivität und Lebensdauer unter Produktionsbedingungen aufweisen.

Insbesondere bestand die Aufgabe, dass dieser Katalysator für die oxidative Veresterung von Methacrolein zu einem Alkylmethacrylat, insbesondere zu MMA geeignet sein soll.

Weitere nicht explizit genannte Aufgaben können sich aus der Beschreibung, den Beispielen, den Ansprüchen oder dem Gesamtzusammenhang der vorliegenden Erfindung ergeben.

### Lösung

Gelöst wurden die gestellten Aufgaben mit Hilfe neuartiger Katalysatorpartikel für die oxidative Veresterung von Aldehyden zu Carbonsäureestern, insbesondere von Methacrolein zu MMA.

Diese erfindungsgemäßen Katalysatoren sind dabei dadurch gekennzeichnet, dass das Katalysatorpartikel die Elemente Sauerstoff, Silizium, Aluminium, ein basisches Element, Gold und mindestens ein Element ausgewählt aus Kobalt, Eisen und Zink, bevorzugt Kobalt enthält. Bevorzugt liegt das Gold dabei elementar in Form von Au{0} und in Form von Nanopartikeln vor. Die anderen Elemente dagegen liegen in oxidierter Form (z.B. als Oxide, Mischoxide, feste Lösungen ineinander usw.) vor.

Ganz besonders bevorzugt besteht das Katalysatorpartikel ausschließlich aus Gold und den Oxiden des Siliziums, Aluminiums, Kobalts und mindestens einem der basischen Elemente. Ein Beispiel für eine besonders geeignete Zusammensetzung weist SiO₂, Al₂O₃, Co₃O₄, MgO und Au auf, insbesondere ausschließlich diese Verbindungen.

Weiterhin sind die erfindungsgemäßen Katalysatorpartikel dadurch charakterisiert, dass die maximale Gold- bzw. die maximale Eisen-, Zink- oder Kobaltkonzentration des Katalysatorpartikels im Außenbereich desselben zu finden ist. Der besagte Außenbereich macht dabei maximal 60 %, bevorzugt maximal 40 % und besonders bevorzugt maximal 30 % des geometrischen Äquivalenzdurchmessers des Katalysatorpartikels aus. Dabei ist die Gold- bzw. Eisen-, Zink- und/oder Kobaltkonzentration in diesem Außenbereich mindestens 1,5 mal, bevorzugt mindestens zweimal und insbesondere bevorzugt mindestens 2,5 mal höher ist als die entsprechende Konzentration dieser Elemente im mittleren Bereich, der den verbliebenen Bereich des geometrischen Äquivalenzdurchmessers des Katalysatorpartikels ausmacht. Besonders bevorzugt befindet sich das Gold zu mehr als 90% in diesem Außenbereich.

Die Ermittlung und Analyse der Verteilung der Konzentrationen von Gold und/oder Eisen-, Zink- und/oder Kobalt entlang des Katalysatorpartikelprofils kann z.B. durch die Einbettung der Partikeln in eine Polymermatrix, nachfolgende Polierung und anschließende REM-EDX Analyse erfolgen. Eine analoge Analysemethode mittels Röntgen Microprobe (EPMA) wird z.B. in EP 2 210 664 A1 auf Seite 18 beschrieben.

Bei den basischen Elementen handelt es sich insbesondere um ein Alkalimetall (Li, Na, K, Rb, Cs, Fr), ein Erdalkalimetall (Be, Mg, Ca, Sr, Ba), ein Seltenerdemetall (Sc, Y, La, Ce. Pr, Nd, Pm, Sm, Eu, Gd, Tb, Dy, Ho, Er, Tm, Yb, Lu) oder um Mischungen aus diesen Metallen. Das basische Element liegt dabei in der Regel auch als Oxid vor.

Überraschenderweise hat sich gezeigt, dass solche erfindungsgemäßen Katalysatorpartikel, enthaltend Au und Eisen-, Zink- und/oder Kobalt im Außenbereich einer Egg-Shell-Struktur, die oben diskutierten Probleme lösen. Dies gilt insbesondere in Bezug auf den Erhalt einer hohen Aktivität und gleichzeitig Selektivität des für die oxidative Veresterung eingesetzten Katalysatorpartikels über eine lange Zeit. Damit weisen die erfindungsgemäßen Katalysatorpartikel eine besonders gute Kombination aus a) einem geringen mechanischen Abrieb des Katalysatorpartikels, b) einem geringen Leaching von Metallionen, die beispielsweise im Falle von Eisen in einem erfindungsgemäß hergestellten MMA zu Problemen bezüglich der Stabilität führen können, aus dem Partikel und c) einem langfristigen Erhalt der Katalysatorleistung bzgl. Aktivität und Selektivität.

Insbesondere bevorzugt sind Zusammensetzungen, in denen das Katalysatorpartikel bezogen auf die Gesamtmolmenge von Gold, Silizium, Aluminium, basischen Elementen und Eisen, Zink und/oder Kobalt - also ohne Berücksichtigung weiterer Elemente, insbesondere des Sauerstoffs - 0,03 bis 3 mol%, bevorzugt 0,1 bis 2 mol% Gold, 40 bis 90 mol%, bevorzugt 65 bis 85 mol% Silizium, 3 bis 40 mol%, bevorzugt 5 bis 30 mol% Aluminium, 2 bis 40 mol%, bevorzugt 5 bis 30 mol% des basischen Elements und 0,1 bis 20 mol%, bevorzugt 0,5 bis 15 mol% Eisen, Zink und/oder Kobalt enthält, wobei das Molverhältnis von Eisen, Zink und Kobalt zu Gold insgesamt zwischen 0,1 und 20, bevorzugt zwischen 1 und 15 beträgt. Wie bereits aufgeführt liegen alle dieser Elemente außer dem Gold dabei in der Regel als Oxide vor.

Alle Anteile, vor allem der Anteil an basischem Element, beziehen sich auf einen frisch hergestellten Katalysator. Dieser Katalysator kann in einem weiteren Schritt optional mit einer Säure behandelt werden, so dass der Anteil an basischem Element sich von ursprünglichen 2 bis 40 mol% auf 0,01 bis 30 mol% reduziert. Zusätzlich kann ein solcher frisch hergestellte Katalysator während eines Prozesses zur Herstellung von Carbonsäureestern einen Teil des basischen Elements verlieren. Dies kann je nach Art des Katalysators sowohl zu einer geringfügigen Verbesserung als auch zu einer Verschlechterung der Aktivität und/oder Selektivität des Prozesses führen kann.

Die aufgeführten Mengenangaben der Elemente Si, Al, Au, und der basischen Elemente, sowie Fe, Zn und/oder Co beziehen sich bevorzugt auf 100 mol% der Zusammensetzung des Katalysators, ohne dass dabei Sauerstoff einbezogen wäre. Diese Angabe der Zusammensetzung unter nicht Berücksichtigung des in den Oxiden vorhandenen Sauerstoffs ist zweckmäßig, da einige der Elemente deutlich unterschiedliche Oxidationsstufen aufweisen bzw. beispielsweise auch Mischoxide vorliegen können. Bevorzugt besteht der Katalysator mit Ausnahme des Sauerstoffs aus den angegebenen Elementen.

Bevorzugt weisen die Katalysatorpartikel einen mittleren geometrischen Äquivalenzdurchmesser zwischen 1 und 1000 µm, bevorzugt 10 und 250 µm und besonders bevorzugt zwischen 25 und 200 µm auf. Die Dicke des Außenbereichs liegt dabei bevorzugt zwischen 2 und 100 µm, bevorzugt zwischen 5 und 50 µm. Die Größe des geometrischen Äquivalenzdurchmessers wird dabei angegeben, da die Partikel nicht zwingend gänzlich sphärisch vorliegen müssen, sondern durchaus auch komplexere Formen aufweisen können. Bevorzugt liegen die Partikel jedoch nahezu oder ideal-sphärisch vor.

Auch sei darauf hingewiesen, dass die so betrachtete Grenze zwischen dem Kern und einer Schale weiterhin nicht scharf sein wird, sondern insbesondere in Form eines Gradienten mit sich ändernder Zusammensetzung vorliegen kann. So kann beispielsweise die Konzentration an Goldnanopartikeln vom Kern aus gesehen von innen nach außen zunehmen. Dies ergibt sich allein schon aus der Tatsache, dass die erfindungsgemäßen Partikel in der Regel eine Porosität aufweisen.

Ein beispielhafter Wert von 80 µm Dicke des Außenbereichs, z.B. bei einem Partikel mit einem Äquivalenzdurchmesser von 200 µm bedeutet im Idealfall, dass sich über den Durchmesser gesehen an den beiden Außenenden desselben jeweils 40 µm Außenbereich und dazwischen 120 µm mittlerer Bereich befinden. Diese Größe wurde gewählt um die Egg-Shell-Struktur des erfindungsgemäßen Katalysators zu beschreiben. Die Grenze zwischen Außen- und Innenbereich kann dabei vom Fachmann bei der Untersuchung der Partikel, in den angegeben Bereichen relativ frei gewählt werden. Entscheidend ist erfindungsgemäß, dass sich innerhalb des angegebenen Bereichs eine Grenze findet, an der die Bedingungen bezüglich der Eisen-, Zink- und/oder Kobalt- und der Goldkonzentration gegeben sind. Dies ist in Bezug auf die Eisen, Zink bzw. insbesondere die Kobaltkonzentration der erfinderische Kern der vorliegenden Erfindung.

Bevorzugt liegen Gold und/oder Gold- und Metalloxid-, insbesondere Kobaltoxid-haltige Partikel mit einem mittleren Durchmesser zwischen 1 und 20 nm, bevorzugt 2 und 10 nm im Außenbereich des Katalysatorpartikels vor. Je nach Herstellungsverfahren ist es erfindungsgemäß sowohl möglich, dass das Gold in Form reiner Partikel als auch in einer Mischform z.B. mit dem Kobaltoxid vorliegt. Dabei ist das Gold in letzterem Fall in der Regel nur mit einem Teil des Kobaltoxids gemischt. Weiterhin ist es in beiden Ausführungsformen optional auch möglich, dass die Gold- bzw. goldhaltigen Partikel zur Stabilisierung zusätzlich mit einer dünnen Schicht, z.B. aus SiO₂ und/oder Al₂O₃ versehen werden.

Bevorzugt sind die erfindungsgemäßen Katalysatorpartikel porös. Wobei sich die Porosität in der Regel nicht auf die Gold- bzw. goldhaltigen Phasen bezieht. Dabei weisen solche porösen Katalysatorpartikel eine spezifische Oberfläche zwischen 100 und 300 m²/g, bevorzugt zwischen 150 und 250 m²/g auf. Weiterhin beträgt in der Regel der durchschnittliche Porendurchmesser dabei 1 bis 50 nm, bevorzugt 2 bis 20 nm.

Neben den beschriebenen Katalysatoren für eine oxidative Veresterung ist insbesondere auch ein Verfahren zur Herstellung von Katalysatoren für eine oxidative Veresterung ein Bestandteil der vorliegenden Erfindung. Dieses Verfahren zur Herstellung von Katalysatorpartikeln ist dadurch gekennzeichnet, dass dieses mindestens die folgenden Schritte aufweist:
1) Auftragung mindestens einer Eisen-, Zink- oder Kobaltverbindung auf ein Partikel, bestehend aus den Oxiden des Siliziums, des Aluminiums und optional eines oder mehrerer Alkali-, Erdalkali- oder Seltenerdemetalle,
2) optionale und gleichzeitig bevorzugte partielle oder vollständige Oxidation des Materials aus 1) und optionale Trocknung/Kalzinierung,
3) Auftragung mindestens einer Goldverbindung auf das Material aus Schritt 2) und
4) Trocknung und/oder Kalzinierung des Materials aus Schritt 3).

Die aus den Oxiden des Siliziums, des Aluminiums und optional eines oder mehrerer Alkali-, Erdalkali- oder Seltenerdemetalle bestehende Trägerpartikel können wie z.B. gemäß Verfahren, wie sie in US 6,228,800 B1, Seite 11 oder US 6,040,472, Seite 27 beschrieben sind, hergestellt werden. Dabei können die Silizium-, Aluminium und optional Alkali-, Erdalkali- oder Seltenerdeverbindungen bevorzugt als wässrige Lösungen und/oder wässrige Suspensionen sukzessive oder gleichzeitig miteinander vermischt werden und das so hergestellte Gemisch mindestens einer thermischen Behandlung unterzogen werden. Als bevorzugte Siliziumverbindung kann z.B. Silikasol, d.h. eine Siliziumdioxidnanopartikeldispersion in Wasser dienen. Bevorzugte Aluminium, Alkali-, Erdalkali- oder Seltenerdeverbindungen sind z.B. die entsprechenden wasserlöslichen Nitrate oder Sulfate. Bei Vermischung dieser Verbindungen werden die Temperatur, der pH-Wert und die Rührgeschwindigkeit speziell eingestellt. Bevorzugt verläuft die Vermischung bei einer Temperatur zwischen -20 und 90 °C, besonders bevorzugt zwischen -15 und 50 °C. Der pH-Wert der Mischung kann mittels Zugabe einer Base oder Säure eingestellt werden. Besonders bevorzugt stellt man einen pH Wert zwischen 0,3 und 2,5 oder zwischen 8,0 und 12,0 ein. Eine bevorzugte Rührgeschwindigkeit beträgt zwischen 50 und 1000 UpM. Unter thermischer Behandlung versteht sich eine partielle oder vollständige Entfernung des Wassers und nachfolgende oder gleichzeitige Zersetzung der thermisch labilen Verbindungen wie z.B. Nitrate unter Erhitzung. Bevorzugt erfolgt eine solche thermische Behandlung zweistufig. In einer ersten Stufe wird das Gemisch von Si, Al und Alkali/Ardalkali/Selteerdenmetalle bei Ausgangstemperatur zwischen 95 und 250 °C, bevorzugt zwischen 100 und 150 °C sprühgetrocknet. In einer zweiten Stufe wird das zuvor getrocknete Material bei einer Temperatur von 250 bis 1000 °C, bevorzugt von 280 bis 800 °C kalziniert.

Zu Verfahrensschritt 1) ergeben sich folgende weitere Aspekte: Die Eisen-, Zink- und/oder Kobaltkomponente, bevorzugt nur eine Kobaltkomponente kann durch Imprägnierung - z.B. durch die sogenannte "incipient wetness impregnation" Methode oder andere Imprägnierungsmethoden - auf das Trägermaterial aufgetragen werden. Bevorzugt wird die Eisen, Zink und/oder Kobaltkomponente aber bei Temperatur zwischen 50 und 100 °C, optimaler Weise bei einer Temperatur zwischen 70 und 95 °C aus einer wässrigen Lösung aufgetragen. Weiterhin handelt es sich bei der Eisen-, Zink- und/oder Kobaltverbindung, um eine wasserlösliche Verbindung, die in Form einer wässrigen Lösung zugegeben wird. Durch die Zugabe bei erhöhten Temperaturen wird gleichzeitig der positive Effekt erzielt, dass die Strukturen der Poren im Katalysatorpartikel positiv beeinflusst wird und damit ein insgesamt stabileres Partikel erhalten wird. Andererseits hat sich überraschend gezeigt, dass die chemische Anbindung des Eisens, Zinks und/oder Kobalts an den Träger durch die höhere Temperatur bei der Zugabe verbessert ist. Damit werden die Materialverluste in einem optionalen Waschschritt genauso wie ein späteres Leaching von Metallionen reduziert. Die beschriebene Imprägnierung kann auch bei noch höheren Temperaturen erfolgen mit der Notwendigkeit, dass dann für den Imprägnierungsprozess in diesem Prozessschritt der Katalysatorherstellung unter Druck gearbeitet werden muss.

Die Oxidation in Verfahrensschritt 2) erfolgt bevorzugt durch das Erhitzen der Partikel oder der die Partikel enthaltenden Suspension aus Verfahrensschritt 1) in Gegenwart von Sauerstoff, z.B. in Form von Luft. Alternativ kann die Oxidation durch Zugabe eines Oxidationsmittels z.B. in die Suspension erfolgen. Bei dem Oxidationsmittel kann es sich beispielsweise um H₂O₂ handeln.
Bei der Oxidation werden insbesondere z.B. Co(II) Verbindungen zu Co(III) teilweise oder vollständig oxidiert. Wenn man stattdessen Gold(III) Verbindungen, bei denen es sich um ein starkes Oxidationsmittel handelt, in Gegenwart von Co(II) Verbindungen einsetzt, kann es zu unerwünschter frühen Reduktion von Gold(III) zu metallischem Gold führen, was wiederum zu einem inaktiven oder weniger aktiven Oxidationskatalysator führen kann. Damit ist insbesondere dieses Vorgehen weniger bevorzugt.
Der Verfahrensschritt 2) kann optional eine thermische Behandlung, wie z.B. eine weitere Trocknung und/oder Kalzinierung, beinhalten, die vor, während oder nach dem Auftragen von Kobalt erfolgen kann.

Die bevorzugte Oxidation in Verfahrensschritt 2 erfolgt durch eine Kalzinierung in Gegenwart von Sauerstoff bei mindestens 200 °C.

Verfahrensschritt 3) erfolgt durch Auftragung mindestens einer Goldkomponente, insbesondere in ionischer Form auf das Material aus Verfahrensschritt 2) und eine anschließende thermische Behandlung in Verfahrensschritt 4), z.B. in Form einer Kalzinierung. Insbesondere erfolgt das Auftragen des Golds mit einer sauren goldhaltigen Lösung, welche in der Regel einen pH-Wert zwischen 0,5 und 5, optimaler Weise zwischen 1 und 4 aufweist. Eine solche Lösung wird mit Goldsäure hergestellt. Bevorzugt wird diese Lösung anschließend teilweise oder vollständig neutralisiert, so dass der pH-Wert des resultierenden Gemischs zwischen 2 und 8, bevorzugt zwischen 3 und 7 liegt. Dies erfolgt durch Zugabe einer Base, z.B. in Form einer NaOH-Lösung. Zu der Variante der Basenzugabe gibt es wiederum zwei alternative Ausführungsformen:
In der ersten Ausführungsform wird in Verfahrensschritt 3) zu einer wässrigen Suspension das Partikel aus Verfahrensschritt 2) zuerst die basische Lösung und anschließend die Lösung, enthaltend Goldsäure, mit einem pH-Wert zwischen 0,5 und 5 gegeben.

In der zweiten Ausführungsform wird in Verfahrensschritt 3) zu einer wässrigen Suspension der Partikel aus Verfahrensschritt 2) eine Lösung gegeben, die dadurch erhalten wurde, dass die Goldsäure-Lösung, zuvor aufweisend einen pH-Wert zwischen 0,5 und 5, durch die Zugabe einer Base, bevorzugt einer NaOH-Lösung, teilweise oder vollständig neutralisiert wurde.

Die Azidität der goldhaltigen Lösung bzw. der pH Wert der goldhaltigen Lösung hat vor allem einen großen Einfluss auf die Ausbildung diverser Spezies von Gold(III)-Komplexionen in der Lösung (siehe dazu beispielsweise Geochimica et Cosmochimica Acta Vol. 55, pp. 671-676) und letztendlich auf die Natur der Bindung an die Oberfläche des Trägers

Beide Kalzinierungsschritte in Verfahrensschritten 2) und 4) werden bevorzugt in einer Oxidationsatmosphäre, z.B. in Gegenwart von Luftsauerstoff, durchgeführt. Die Verfahrensschritte 1) und 3), sowie optional Verfahrensschritt 2) werden insbesondere in einem wässrigen Medium, insbesondere in einer wässrigen Suspension der Partikel durchgeführt.

Bevorzugt werden die Oxide des Siliziums, des Aluminiums und optional eines oder mehrerer Alkali-, Erdalkali- oder Seltenerdemetalle innerhalb der Verfahrensschritte 1) bis 3), bevorzugt in oder direkt nach Verfahrensschritt 1) mindestens einer thermischen Behandlung mit Wasser unterzogen. Dabei beträgt die Wassertemperatur zwischen 50 und 100 °C, bevorzugt zwischen 70 und 95 °C. Durch diese thermische Behandlung wird der positive Effekt erzielt, dass die Strukturen der Poren im Katalysatorpartikel positiv beeinflusst werden und damit ein insgesamt stabileres Partikel erhalten wird.

In einer besonderen, jedoch nicht unbedingt bevorzugten Variante dieses Verfahrens enthält die goldhaltige Lösung neben der Goldverbindung mindestens eine zusätzliche Verbindung. Diese Verbindung weist wiederum die Elemente Silizium, Aluminium und das oder die basischen Elemente des Trägermaterials in ionischer Form auf. Mit dieser Variante kann eine zusätzliche Schutzschicht für die Goldnanopartikel entstehen, die für höhere Standzeit des Katalysators zusätzlich förderlich ist. Silizium in ionischer Form bedeutet in diesem Fall, dass Silikate, z.B. Natriumsilikate oder Ammoniumsilikate, die später bei einem optionalen thermischen Sintern oder Kalzinieren zu Siliziumoxiden umgesetzt werden, in der Lösung enthalten sind. Die anderen Elemente in ionischer Form bedeuten die entsprechenden wasserlöslichen Salze, z.B. Aluminiumnitrat, Aluminiumsulfat usw.

Nach der beschriebenen Herstellung der Partikel, einem Isolieren, z.B. mittels Filtration, und weiterem Reinigen, werden diese abschließend besonders bevorzugt in Verfahrensschritt 4) kalziniert. Dies kann beispielsweise bei Temperaturen zwischen 200 und 1000 °C, besonders bevorzugt zwischen 250 und 800 °C geschehen.

Überaschenderweise wurde gefunden, dass diese Art der Auftragung von Gold- und Eisen-, Zink- und/oder Kobalt auf einen vorher hergestellten oxidischen Träger besonders vorteilhaft ist. Besonders bevorzugt ist dabei die diese Art der Auftragung für die Kombination aus Gold und Kobalt. Dies gilt insbesondere im Vergleich zu einer aus dem Stand der Technik bekannten Methode, bei der Herstellung vom Trägermaterial aus dem Gemisch von Co (Fe, Zn), Si, Al Verbindungen und den Salzen von basischen Elementen gleichzeitig erfolgt. Damit wird es ermöglicht, einen aktiven und selektiven Katalysator für die oxidative Veresterung von Aldehyden zu Carbonsäureestern, wie z.B. von Methacrolein zu MMA herzustellen. Besonders vorteilhaft ist es dabei anscheinend, dass der getrocknete und kalzinierte, poröse oxidische Träger in Verfahrensschritt 1 mit der Lösung einer löslichen Eisen-, Zink- und/oder Kobaltverbindung behandelt wird. Damit wird bewirkt, dass die Eisen-, Zink- und/oder Kobaltionen bevorzugt die Oberfläche des Trägers, u.a. die Porenoberfäche im Außenbereich, belegen und anschließend dort durch das Trocknen und/oder Kalzinieren fixiert werden. Wichtig bei diesem Verfahren ist die bevorzugte Belegung der Porenoberfläche mit den Eisen-, Zink- und/oder Kobaltionen - und nicht die homogene Verteilung dieser, wie sie im Stand der Technik, vor allem in US 2013/0172599 beschrieben ist. Die Kombination mit dem gleichfalls an der Oberfläche des Katalysatorpartikels angereicherten Gold führt dazu, dass die beiden katalytisch aktiven Komponenten Co (Fe, Zn) und Au dem Reaktionsmedium besser zugänglich sind, sich besser gegenseitig aktivieren können und gleichsam der Katalysator überraschend stabil und langlebig ist.

Außerdem wurde überraschend gefunden, dass infolge einer sukzessiven Auftragung der Eisen, Zink und/oder Kobalt- und der Goldverbindung, kombiniert mit einer entsprechenden oxidativen thermischen Behandlung, kontrollierte Übergänge zwischen den verschiedenen Oxidationsstufen des Goldes - typischerweise von Au³⁺ zu Au⁰ - und des Kobalts - typischerweise von Co²⁺ zu Co^{x+}, mit 2 < X ≤ 3 - an der Oberfläche des oxidischen Trägers durchläuft. Für Eisen liegen typischerweise Oxidationsstufen von Fe²⁺ zu Fe^{x+} mit 2 < X ≤ 3 vor. Für Zink ist nur die Oxidationsstufe (II) relevant. Entsprechend findet hier keine Oxidation des Zinks statt.

Diese nachträgliche Redoxreaktion führt zu den aktiveren und selektiveren Katalysatoren im Vergleich zu den aus dem Stand der Technik bekannten Varianten (siehe dazu die unten folgenden Vergleichsbeispiele).

Die Kombination aus diesen Faktoren ermöglicht es, einen Katalysator mit Schalenstruktur der aktiven Komponenten (z.B. mit Co und Au) zu synthetisieren, der hohe Aktivität, Selektivität und lange Standzeiten aufweist.

Neben den beschriebenen Katalysatoren und dem beschriebenen Verfahren zu deren Herstellung ist auch die Verwendung eines solchen erfindungsgemäßen, bzw. erfindungsgemäß hergestellten Katalysators bei der Umsetzung von Aldehyden mit Sauerstoff und einem Alkohol zu einem Carbonsäureester, insbesondere von (Meth)acrolein mit Sauerstoff und einem monofunktionellen Alkohol zu einem Alkyl(meth)acrylat ein Bestandteil der vorliegenden Erfindung. Die Klammer in (Meth)acrolein bedeutet dabei, dass es sich bei diesem Rohstoff sowohl um Acrolein als auch um Methacrolein handeln kann. Entsprechend bedeutet Alkyl(meth)acrylat sowohl Alkylacrylat, als auch Alkylmethacrylat. Dabei wird diese Alkylgruppe durch den eingesetzten Alkohol bestimmt.

Bevorzugt wird bei dieser Verwendung Methacrolein in Anwesenheit des erfindungsgemäßen Katalysators mit Sauerstoff und Methanol zu MMA umgesetzt.

Alternativ kann bei dieser Verwendung auch (Meth)acrolein mit Sauerstoff und einem di-, tri- oder tetrafunktionellen Alkohol zu einem Hydroxyalkyl(meth)acrylat und di-, tri- bzw. tetra-(Meth)acrylat umgesetzt werden. Die letzteren Verbindungen sind als Vernetzer bekannt. Ein besonders bevorzugtes Beispiel für einen di-funktionellen Alkohol ist Ethylenglycol.

Besonders bevorzugt wird die oxidative Veresterung in Anwesenheit des erfindungsgemäßen Katalysators kontinuierlich durchgeführt. Ganz besonders bevorzugt wird der Katalysator während der oxidativen Veresterung in einem gerührten Reaktor in Suspensionsform (als Slurry) angewendet.

### Beispiele

Die Analyse der Konzentrationsprofile für Co und Au innerhalb der Katalysatorpartikel erfolgt mittels der REM-EDX Line Scan Methode. Dabei wurde folgender Setup verwendet:
Mikroskop: Jeol JSM 7600F; Analyse - Oxford AZtec mit X-Max 150 Detektor.

Die Proben wurden in einem Harz eingebettet und mit einem Leica Ultramikrotom an einem Diamantmesser angeschnitten.
Die Analysenmethode basiert auf EDX bei 20 KV Beschleunigungsspannung. Ausgewertet wurden der Co K-alpha Peak bei 6,924 KeV und Au M-alpha Peak bei 2,120 KeV

Die folgenden Beispiele dokumentieren den Effekt hauptsächlich für Kobalt-haltige Katalysatoren. Auch konnte der Effekt für Zn-haltige Katalysatoren gezeigt werden. Die Ergebnisse sind einfach auf Katalysatoren mit Eisen oder Mischungen aus zwei oder drei Elemente ausgewählt aus Eisen, Zink und Kobalt übertragbar.

### Beispiel 1 (SiO₂-Al₂O₃-MgO)

In einem 250 mL Becherglas werden 21,36 g Mg(NO₃)₂*6H₂O, 31,21 g Al(NO₃)₃*9H₂O zusammen vorgelegt und in 41,85 g VE Wasser unter Rühren mit einem Magnetrührer gelöst. Danach werden 1,57 g 60%ige HNO₃ unter Rühren zugegeben. 166,67 g Silicasol (Köstrosol 1530AS von der Firma Bad Köstritz, 30 Gew% SiO₂, Mittelgroße der Partikel: 15 nm) werden in einen 500 mL Dreihalskolben eingewogen und unter Rühren auf 15 °C gekühlt. 2,57 g 60%ige HNO₃ werden unter Rühren langsam zum Sol zugegeben. Bei 15 °C wird die Nitratlösung innerhalb von 45 min zum Sol unter Rühren zugegeben. Nach der Zugabe wird das Gemisch innerhalb von 30 min auf 50 °C erhitzt und weitere 24 h bei dieser Temperatur gerührt. Nach dieser Zeit wird das Gemisch bei 130 °C Ausgangstemperatur sprühgetrocknet. Das getrocknete Pulver (sphärisch, mittlere Partikelgroße 60 µm) wird in dünner Schicht im Naberofen innerhalb von 2h auf 300 °C erhitzt, 3 h bei 300 °C gehalten, innerhalb von 2 h auf 600 °C erhitzt und schließlich für 3 h bei 600 °C gehalten.

### Beispiel 2

Eine Suspension von 10 g SiO₂-Al₂O₃-MgO des Trägers aus Beispiel 1 in 33,3 g VE-Wasser wird auf 90 °C aufgeheizt und 15 min bei dieser Temperatur gerührt. Zu dieser Suspension wird unter Rühren eine vorher auf 90 °C erhitzte Lösung von Co(NO₃)2*6H₂O (569 mg, 1,95 mmol) in 8,3 g Wasser zugegeben. Nach der Zugabe wurde das Gemisch weitere 30 min bei 90 °C gerührt, dann abgekühlt, bei Raumtemperatur abfiltriert und schließlich sechsmal mit jeweils 50 mL Wasser gewaschen. Das Material wurde 10 h bei 105 °C getrocknet, fein gemörsert dann innerhalb von 1h von 18 auf 450 °C aufgeheizt und darauf 5h bei 450 °C kalziniert.

### Beispiel 3

Eine Suspension von 15 g SiO₂-Al₂O₃-MgO des Trägers aus Beispiel 1 in 50 g VE-Wasser wird auf 90 °C aufgeheizt und 15 min bei dieser Temperatur gerührt. Zu dieser Suspension wird unter Rühren eine vorher auf 90 °C erhitzte Lösung von CoCl₂ (697 mg, 2,93 mmol) und LiCl (1,24 g) in 12,5 g Wasser zugegeben. Nach der Zugabe wurde das Gemisch weitere 30 min bei 90 °C gerührt, dann abgekühlt, bei Raumtemperatur abfiltriert und schließlich sechsmal mit jeweils 50 mL Wasser gewaschen. Das Material wurde 10 h bei 105 °C getrocknet, fein gemörsert dann innerhalb von 1h von 18 auf 450 °C aufgeheizt und darauf 5h bei 450 °C kalziniert.

### Beispiel 4

Eine Suspension von 10 g eines mit Kobalt dotierten SiO₂-Al₂O₃-MgO-Trägers aus Beispiel 2 in 33,3 g VE-Wasser wird auf 90 °C aufgeheizt und 15 min bei dieser Temperatur gerührt. Zu dieser Suspension wird unter Rühren eine vorher auf 90 °C erhitzte Lösung von HAuCl₄*3H₂O (205 mg) in 8,3 g Wasser zugegeben. Nach der Zugabe wurde das Gemisch weitere 30 min bei 90 °C gerührt, dann abgekühlt, bei Raumtemperatur abfiltriert und schließlich sechsmal mit jeweils 50 mL Wasser gewaschen. Das Material wurde 10 h bei 105 °C getrocknet, fein gemörsert dann innerhalb von 1 h von 18 auf 450 °C aufgeheizt und darauf 5h bei 450 °C kalziniert.

Eine Line Scan REM-EDX Analyse von ins Polymermatrix eingebetteten und angeschliffenen Katalytaorpartikeln zeigte eine egg-shell Verteilung für Kobalt und für Gold im Partikel:

### Beispiel 5

Eine Suspension von 10 g eines mit Kobalt dotierten SiO₂-Al₂O₃-MgO-Trägers aus Beispiel 2 in 33,3 g VE-Wasser wird auf 90 °C aufgeheizt und 15 min bei dieser Temperatur gerührt. Zu dieser Suspension wird unter Rühren eine vorher auf 90 °C erhitzte Lösung von HAuCl₄*3H₂O (205 mg) und 0,52 mL einer einmolaren NaOH-Lösung (Au/Na = 1:1 mol/mol) in 8,3 g Wasser zugegeben. Nach der Zugabe wird das Gemisch weitere 60 min gerührt, dann abgekühlt, bei Raumtemperatur abfiltriert und schließlich sechsmal mit jeweils 50 mL Wasser gewaschen. Das Material wurde 10 h bei 105 °C getrocknet, fein gemörsert dann innerhalb von 1h von 18 auf 450 °C aufgeheizt und darauf 5h bei 450 °C kalziniert.

Eine Line Scan REM-EDX Analyse von ins Polymermatrix eingebetteten und angeschliffenen Katalytaorpartikeln zeigte eine egg-shell Verteilung für Kobalt und für Gold im Partikel:

### Beispiel 6

Eine Suspension von 10 g eines mit Kobalt dotierten SiO₂-Al₂O₃-MgO-Trägers aus Beispiel 2 in 33,3 g VE-Wasser wird auf 90 °C aufgeheizt und 15 min bei dieser Temperatur gerührt. Diese Suspension wird unter Rühren mit eine vorher auf 90 °C erhitzte Lösung von 0,52 mL einer einmolaren NaOH-Lösung versetzt. Nach 30 min Rühren bei 90 °C wird HAuCl₄*3H₂O (205 mg) in 4,3 g Wasser zugegeben. Nach der Zugabe wurde das Gemisch weitere 60 min gerührt, dann abgekühlt, bei Raumtemperatur abfiltriert und schließlich sechsmal mit jeweils 50 mL Wasser gewaschen. Das Material wurde 10 h bei 105 °C getrocknet, fein gemörsert dann innerhalb von 1 h von 18 auf 450 °C aufgeheizt und darauf 5h bei 450 °C kalziniert.
Eine Line Scan REM-EDX Analyse von ins Polymermatrix eingebetteten und angeschliffenen Katalytaorpartikeln zeigte eine egg-shell Verteilung für Kobalt und für Gold im Partikel:

### Beispiel 7

Eine Suspension von 10 g eines mit Kobalt dotierten SiO₂-Al₂O₃-MgO-Trägers aus Beispiel 3 in 33,3 g VE-Wasser wird auf 90 °C aufgeheizt und 15 min bei dieser Temperatur gerührt. Zu dieser Suspension wird unter Rühren eine vorher auf 90 °C erhitzte Lösung von HAuCl₄*3H₂O (205 mg) und 0,52 mL einer einmolaren NaOH-Lösung (Au/Na = 1:1 mol/mol) in 8,3 g Wasser zugegeben. Nach der Zugabe wird das Gemisch weitere 60 min gerührt, dann abgekühlt, bei Raumtemperatur abfiltriert und schließlich sechsmal mit jeweils 50 mL Wasser gewaschen. Das Material wurde 10 h bei 105 °C getrocknet, fein gemörsert dann innerhalb von 1h von 18 auf 450 °C aufgeheizt und darauf 5h bei 450 °C kalziniert.

Eine Line Scan REM-EDX Analyse von ins Polymermatrix eingebetteten und angeschliffenen Katalysatorpartikeln zeigte eine egg-shell Verteilung für Kobalt und für Gold im Partikel:

### Beispiel 8

Eine Suspension von 10 g des SiO₂-Al₂O₃-MgO-Träger aus Beispiel 1 in 33,3 g VE Wasser wird auf 90 °C aufgeheizt und 15 min bei dieser Temperatur gerührt. Zu dieser Suspension wird unter Rühren eine vorher auf 90 °C erhitzte Lösung von Co(NO₃)₂*6H₂O (569 mg, 1,95 mmol) in 4,2 g Wasser zugegeben. Nach der Zugabe wurde das Gemisch weitere 60 min bei 90 °C gerührt, wobei Luftstrom in die Lösung eingeperlt wird. Nach 60 Min Rühren wird zu dieser Suspension unter Rühren eine vorher auf 90 °C erhitzte Lösung von HAuCl₄*3H₂O (205 mg) in 4,2 g Wasser zugegeben. Nach der Zugabe wurde das Gemisch weitere 30 min bei 90 °C gerührt, dann abgekühlt, bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und für 5h bei 450 °C kalziniert.
Eine Line Scan REM-EDX Analyse von ins Polymermatrix eingebetteten und angeschliffenen Katalysatorpartikeln zeigte eine egg-shell Verteilung für Co und für Gold:

### Beispiel 9

Eine Suspension von 10 g des SiO₂-Al₂O₃-MgO-Trägers aus Beispiel 1 in 33,3 g VE Wasser wird auf 90 °C aufgeheizt und 15 min bei dieser Temperatur gerührt. Zu dieser Suspension wird unter Rühren eine vorher auf 90 °C erhitzte Lösung von Co(NO₃)₂*6H₂O (569 mg, 1,95 mmol) in 4,2 g Wasser zugegeben. Nach der Zugabe wurde das Gemisch weitere 30 min bei 90 °C gerührt. H₂O₂ (30 wt%, 0,45 g) wurde zum Gemisch tropfenweise zugegeben. Nach 60 min weiteren Rührens wird zur Suspension unter Rühren eine vorher auf 90 °C erhitzte Lösung von HAuCl₄*3H₂O (205 mg) in 4,2 g Wasser zugegeben. Nach der Zugabe wurde das Gemisch weitere 30 min bei 90 °C gerührt, dann abgekühlt, bei Raumtemperatur filtriert und anschließend sechsmal mit jeweils 50 mL Wasser gewaschen. Das Material wurde 10 h bei 105 °C getrocknet, fein gemörsert, dann innerhalb von 1h von 18 auf 450 °C aufgeheizt und bei 450 °C für 5h kalziniert.

Eine Line Scan REM-EDX Analyse von ins Polymermatrix eingebetteten und angeschliffenen Katalysatorpartikeln zeigte eine egg-shell Verteilung für Co und für Gold.

### Beispiel 10

Co(NO₃)₂*6H₂O (569 mg, 1,95 mmol) wird in 5 g VE Wasser gelöst und die Lösung mit 10 g eines SiO₂-Al₂O₃-MgO-Trägers aus dem Beispiel 1 unter intensivem Schütteln vermischt. Der so erhaltene trocken aussehende Träger wurde in einer dünnen Schicht im Trockenschrank bei 105 °C 10h getrocknet, dann fein gemörsert und in dünner Schicht im Naberofen innerhalb von 2h auf 300 °C erhitzt, 3h bei 300 °C gehalten, innerhalb weiterer 2h auf 600 °C erhitzt und schließlich 3h bei 600 °C gehalten.

### Beispiel 11

Eine Suspension von 10 g des mit Kobalt dotierten SiO₂-Al₂O₃-MgO-Trägers aus Beispiel 8 in 33,3 g VE Wasser wird auf 90 °C aufgeheizt und 15 min bei dieser Temperatur gerührt. Zu dieser Suspension wird unter Rühren eine vorher auf 90 °C erhitzte Lösung von HAuCl₄*3H₂O (205 mg) in 8,3 g Wasser zugegeben. Nach der Zugabe wurde das Gemisch weitere 30 min gerührt, dann abgekühlt, bei Raumtemperatur filtriert und anschließend sechsmal mit jeweils 50 mL Wasser gewaschen. Das Material wurde 10 h bei 105 °C getrocknet, fein gemörsert, dann innerhalb von 1h von 18 auf 450 °C aufgeheizt und bei 450 °C für 5h kalziniert.
Eine Line Scan REM-EDX Analyse von ins Polymermatrix eingebetteten und angeschliffenen Katalytaorpartikeln zeigte eine egg-shell Verteilung für Co und für Gold.

### Beispiel 12

Zn(NO3)₂*6H₂O (580 mg, 1,95 mmol) wird in 5 g VE Wasser gelöst und die Lösung mit 10 g eines SiO₂-Al₂O₃-MgO-Trägers aus dem Beispiel 1 unter intensivem Schütteln vermischt. Der so erhaltene trocken aussehende Träger wurde in einer dünnen Schicht im Trockenschrank bei 105 °C 10h getrocknet, dann fein gemörsert und in dünner Schicht im Naberofen innerhalb von 2h auf 300 °C erhitzt, 3h bei 300 °C gehalten, innerhalb weiterer 2h auf 600 °C erhitzt und schließlich 3h bei 600 °C gehalten.

### Beispiel 13

Eine Suspension von 10 g des mit Zink dotierten SiO₂-Al₂O₃-MgO-Trägers aus Beispiel 12 in 33,3 g VE Wasser wird auf 90 °C aufgeheizt und 15 min bei dieser Temperatur gerührt. Zu dieser Suspension wird unter Rühren eine vorher auf 90 °C erhitzte Lösung von HAuCl₄*3H₂O (205 mg) in 8,3 g Wasser zugegeben. Nach der Zugabe wurde das Gemisch weitere 30 min gerührt, dann abgekühlt, bei Raumtemperatur filtriert und anschließend sechsmal mit jeweils 50 mL Wasser gewaschen. Das Material wurde 10 h bei 105 °C getrocknet, fein gemörsert, dann innerhalb von 1h von 18 auf 450 °C aufgeheizt und bei 450 °C für 5h kalziniert.
Eine Line Scan REM-EDX Analyse von ins Polymermatrix eingebetteten und angeschliffenen Katalytaorpartikeln zeigte eine egg-shell Verteilung für Zn und für Gold.

### Beispiele 14 bis 21 (Batchtests zur MMA-Herstellung)

Ein Gold-haltiger Katalysator gemäß Tabelle 1 (384 mg), Methacrolein (1,20 g) und Methanol (9,48g) wurden 2h bei 60 °C und 30 bar Druck in einer Atmosphäre von 7 vol% O₂ in N₂ in einem 140 mL Stahlautoklav mit Magnetrüher gerührt. Nach 2h wurde das Gemisch abgekühlt, entgast, filtriert und mittels GC analysiert. Jeder Katalysator wurde mindestens zweimal unter identischen Bedingungen getestet, die Ergebnisse der jeweiligen Experimente gemittelt. Der resultierenden Umsatz von Methacrolein (U(MAL), %), die Raum-Zeit-Ausbeute (RZA, mol MMA/kg Kat h) und die Selektivität zu MMA (S(MMA), %) für jeden getesteten Katalysator sind in der folgenden Tabelle 1 zusammengefasst.

**Tabelle 1**

| **Beispiel** | **Kat Bsp.** | **Co (bzw. Zn) Verteilung** | **U(MAL) %** | **RZA, mol MMA/kg(Kat) h** | **S(MMA) %** |
|---|---|---|---|---|---|
| **14** | 4 | Egg-shell | 67,7 | 13,4 | 94,8 |
| **15** | 5 | Egg-shell | 64,6 | 12,5 | 94,3 |
| **16** | 6 | Egg-shell | 64,6 | 12,8 | 96,6 |
| **17** | 7 | Egg-shell | 59,6 | 11,6 | 95,5 |
| **18** | 8 | Egg-shell | 56,6 | 10,4 | 91,4 |
| **19** | 9 | Egg-shell | 56 | 10,6 | 94,3 |
| **20** | 11 | Egg-shell | 58,7 | 11,9 | 92,3 |
| **21** | 13 | Egg-shell | 47,1 | 9,9 | 93,5 |

### Vergleichsbeispiel 1

Eine Suspension von 10 g des dotierten SiO₂-Al₂O₃-MgO-Trägers aus Beispiel 1 in 33,3 g VE Wasser wird auf 90 °C aufgeheizt und 15 min bei dieser Temperatur gerührt. Zu dieser Suspension wird unter Rühren eine vorher auf 90 °C erhitzte Lösung von HAuCl₄*3H₂O (205 mg) in 8,3 g Wasser zugegeben. Nach der Zugabe wurde das Gemisch weitere 30 min gerührt, dann abgekühlt, bei Raumtemperatur filtriert und anschließend sechsmal mit jeweils 50 mL Wasser gewaschen. Das Material wurde 10 h bei 105 °C getrocknet, fein gemörsert, innerhalb von 1 h von 18 auf 450 °C aufgeheizt und bei 450 °C für 5h kalziniert.

### Vergleichsbeispiel 2

In einem 250 mL Becherglas werden 21,35 g Mg(NO₃)₂*6H₂O, 31,21 g Al(NO₃)₃*9H₂O und 4,72 g Co(NO₃)₂*6H₂O zusammen vorgelegt und in 41,85 g VE Wasser unter Rühren auf einem Magnetrührer gelöst. Danach werden 1,57 g 60%ige HNO₃ unter Rühren zugegeben. 166,67 g Silicasol (Köstrosol 1530AS von der Firma Bad Köstritz) werden in einen 500 mL Dreihalskolben eingewogen und unter Rühren auf 15 °C gekühlt. 2,57 g 60% HNO₃ werden weiterhin unter Rühren langsam zum Sol zugegeben. Bei 15 °C wird die Nitratlösung innerhalb von 45 min zum Sol unter Rühren zugegeben. Nach der Zugabe wird das Gemisch auf 50 °C innerhalb von 30 min erhitzt und weitere 24h bei dieser Temperatur gerührt. Nach dieser Zeit wird das Gemisch in einem Sprühtrockner mit einer Ausgangtemperatur von 120 °C getrocknet. Das getrocknete Pulver wird in dünner Schicht im Naberofen über 2 h auf 300 °C erhitzt, 3 h bei 300 °C gehalten, innerhalb weiterer 2h auf 600 °C erhitzt und schließlich weitere 3h bei 600 °C gehalten. Das resultierende Material bestand aus runden Partikeln mit einer durchschnittlichen Partikelgröße von ca 60 µm.

### Vergleichsbeispiel 3

Eine Suspension von 10 g mit Kobalt dotierten SiO₂-Al₂O₃-MgO-Träger aus VB2 in 33,3 g VE Wasser wird auf 90 °C aufgeheizt und 15 min bei dieser Temperatur gerührt. Zu dieser Suspension wird unter Rühren eine vorher auf 90 °C erhitzte Lösung von HAuCl₄*3H₂O (205 mg) in 8,3 g Wasser zugegeben. Nach der Zugabe wurde das Gemisch weitere 30 min gerührt, dann abgekühlt, bei Raumtemperatur filtriert und anschließend sechsmal mit jeweils 50 mL Wasser gewaschen. Das Material wurde 10 h bei 105 °C getrocknet, fein gemörsert, dann innerhalb von 1h von 18 auf 450 °C aufgeheizt und bei 450 °C für 5h kalziniert.

Eine Line Scan REM-EDX Analyse von in der Polymermatrix eingebetteten und angeschliffenen Katalytaorpartikeln zeigte eine homogene Verteilung von Co und schwach ausgeprägte inhomogene Verteilung für Gold:

### Vergleichsbeispiel 4

Eine Suspension von 10 g des SiO₂-Al₂O₃-MgO-Trägers aus Beispiel 1 in 33,3 g VE Wasser wird auf 90 °C aufgeheizt und 15 min bei dieser Temperatur gerührt. Zu dieser Suspension wird unter Rühren eine vorher auf 90 °C erhitzte Lösung von HAuCl₄*3H₂O (205 mg) und Ni(NO₃)₂*6H₂O (567 mg, 1,95 mmol) in 8,3 g Wasser zugegeben. Nach der Zugabe wurde das Gemisch weitere 30 min gerührt, dann abgekühlt, bei Raumtemperatur filtriert und anschließend sechsmal mit jeweils 50 mL Wasser gewaschen. Das Material wurde 10 h bei 105 °C getrocknet, fein gemörsert, dann innerhalb von 1h von 18 auf 450 °C aufgeheizt und bei 450 °C für 5h kalziniert.
Eine Line Scan REM-EDX Analyse von ins Polymermatrix eingebetteten und angeschliffenen Katalytaorpartikeln zeigte eine egg-shell Verteilung für Ni und für Gold:

### Vergleichsbeispiel 5

Eine Suspension von 10 g des SiO₂-Al₂O₃-MgO-Trägers aus Beispiel 1 in 33,3 g VE Wasser wird auf 90 °C aufgeheizt und 15 min bei dieser Temperatur gerührt. Zu dieser Suspension wird unter Rühren eine vorher auf 90 °C erhitzte Lösung von HAuCl₄*3H₂O (205 mg) und Co(NO₃)₂*6H₂O (569 mg, 1,95 mmol) in 8,3 g Wasser zugegeben. Nach der Zugabe wurde das Gemisch weitere 30 min gerührt, dann abgekühlt, bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und bei 450 °C für 5h kalziniert.

### Vergleichsbeispiel 6

Eine Suspension von 10 g des SiO₂-Al₂O₃-MgO-Trägers aus Beispiel 1 in 33,3 g VE Wasser wird auf 90 °C aufgeheizt und 15 min bei dieser Temperatur gerührt. Zu dieser Suspension wird unter Rühren eine vorher auf 90 °C gebrachte Lösung von Co(NO₃)₂*6H₂O (569 mg, 1,95 mmol) in 4,2 g Wasser zugegeben. Nach der Zugabe wird das Gemisch weitere 30 min bei 90 °C gerührt. Zu dieser Suspension wird bei Rühren eine vorher auf 90 °C gebrachte Lösung von HAuCl₄*3H₂O (205 mg) in 4,2 g Wasser zugegeben. Nach der Zugabe wurde das Gemisch weitere 30 min bei 90 °C gerührt, dann abgekühlt, bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und bei 450 °C für 5h kalziniert.

### Vergleichsbeispiel 7

Eine Suspension von 10 g des SiO₂-Al₂O₃-MgO-Trägers aus Beispiel 1 in 33,3 g VE Wasser wird auf 90 °C aufgeheizt und 15 min bei dieser Temperatur gerührt. Zu dieser Suspension wird unter Rühren eine vorher auf 90 °C erhitzte Lösung von HAuCl₄*3H₂O (205 mg) in 4,2 g Wasser zugegeben. Nach der Zugabe wurde das Gemisch weitere 30 min bei 90 °C gerührt. Zu dieser Suspension wird unter Rühren eine vorher auf 90 °C erhitzte Lösung von Co(NO₃)₂*6H₂O (569 mg, 1,95 mmol) in 4,2 g Wasser zugegeben. Nach der Zugabe wurde das Gemisch weitere 30 min bei 90 °C gerührt, dann abgekühlt, bei Raumtemperatur filtriert und anschließend sechsmal mit jeweils 50 mL Wasser gewaschen. Das Material wurde 10 h bei 105 °C getrocknet, fein gemörsert, in 1h von 18 auf 450 °C aufgeheizt und bei 450 °C für 5h kalziniert.

### Vergleichsversuch VB8 bis VB13

**Tabelle 2: Batchtests mit Katalysatoren VB1, VB3 bis VB7**

| **Beispiel** | **Kat Bsp.** | **Co (bzw. Ni) Verteilung** | **U(MAL), %** | **RZA, mol MMA/kg(Kat) h** | **S(MMA), %** |
|---|---|---|---|---|---|
| **VB8** | **VB1** | - | 39 | 7,9 | 91,8 |
| **VB9** | **VB3** | Homogen | 42 | 8,5 | 91,6 |
| **VB10** | **VB4** | Egg-shell | 75,6 | 15,5 | 94,2 |
| **VB11** | **VB5** | | 6,5 | 1,2 | 86,2 |
| **VB12** | **VB6** | | 55,9 | 10,9 | 89,1 |
| **vB13** | **VB7** | | 13,8 | 2,3 | 76,1 |

### Kontinuierlicher Test zur Herstellung von MMA (allgemeine Beschreibung)

Der pH-Wert einer 42,5 gew%igen Lösung von MAL in Methanol wird durch die Zugabe von einer ein-gew%igen Lösung NaOH in Methanol unter Rühren auf pH = 7 eingestellt. Diese Lösung wird mit einer konstanten Zugaberate kontinuierlich einem gerührten und begasten Rührkesselreaktor (Begasung mit Luft) unter 10 bar Druck und 80 °C Innentemperatur zugeführt. Gleichzeitig wird in diesen Reaktor mit 20 g Pulverkalalysator so viel ein-gew%ige NaOH-Lösung (in Methanol) zugeführt, dass der Wert pH = 7 im Reaktor konstant blieb. Das Reaktionsgemisch wurde über einen Filter aus dem Reaktor kontinuierlich entnommen. Die Produktproben wurden nach unten angegebener Zeit entnommen und mittels GC analysiert.

**Tabelle 3: Konti-Tests zur MMA-Herstellung mit ausgewählten Katalysatoren**

| **Beispiel Kat** | **TOS [h]** | **U(MAL), %** | **RZA, mol MMA /kg(Kat) h** | **S(MMA), %** | **D50, µm Frischer Kat** | **D50, µm Gebrauchter Kat** |
|---|---|---|---|---|---|---|
| **4** | 100 | 75.2 | 12.4 | 95.8 | | |
| **4** | **2000** | 73.8 | 12.2 | 95.5 | 55,6 | 55,2 |
| **VB4** | 100 | 77.6 | 10.4 | 96.1 | | |
| **VB4** | **2000** | 69.7 | 9.3 | 91.5 | 55,2 | 4,6 |

Die Beispiele, insbesondere gemäß der Ergebnisse in Tabelle 3 zeigen, dass die erfindungsgemäßen Katalysatoren gegenüber dem Stand der Technik bei identischer Anfangsaktivität und -selektivität deutlich längere Standzeiten als die Katalysatoren des Standes der Technik aufweisen.

### Zu den Abbildungen

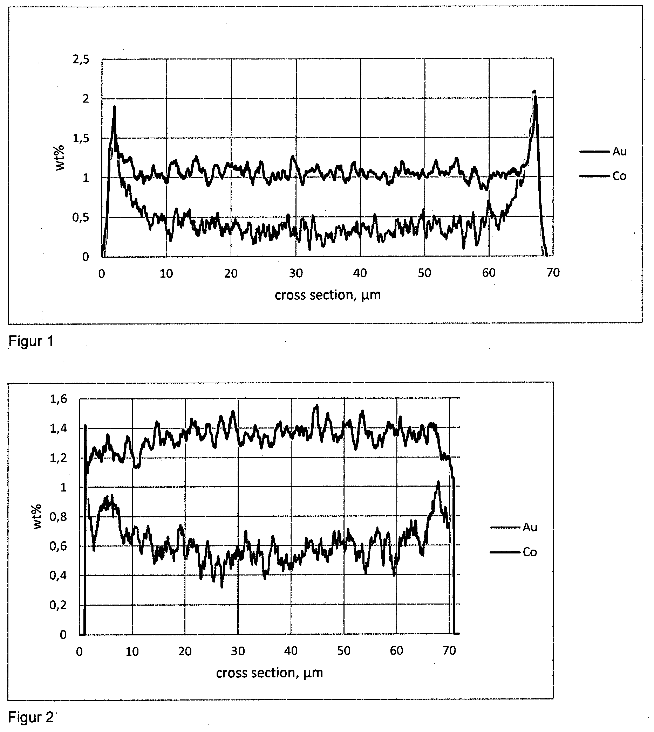

In Figur 1 ist die Verteilung des Goldes (gestrichelte Linie) und des Kobalts (geschlossene Linie) eines geschliffenen Katalysatorpartikel aus Beispiel 4 abgebildet. Man erkennt, die höhere Konzentration beider Metalle in den jeweiligen Außenbereichen der Partikel. Der steile Kurvenanstieg an den äußersten Rändern ist durch eine nicht glatte Oberfläche des Partikels zu erklären.

In Figur 2 ist die Verteilung des Goldes (gestrichelte Linie) und des Kobalts (geschlossene Linie) eines geschliffenen Katalysatorpartikel aus Vergleichsbeispiel 3 abgebildet. Man erkennt, die gleichmäßige Konzentration beider Metalle über dem gesamten Partikel. Der steile Kurvenanstieg an den äußersten Rändern ist durch eine nicht glatte Oberfläche des Partikels zu erklären.

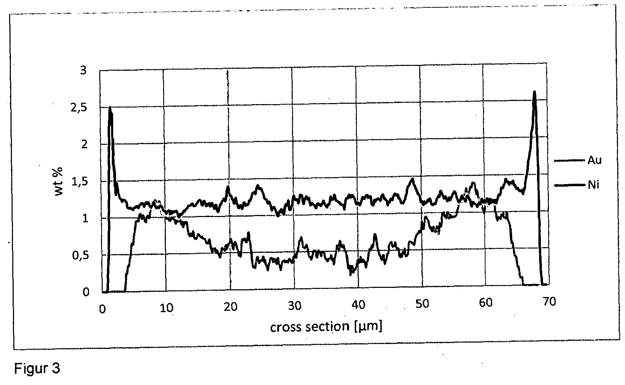

In Figur 3 ist die Verteilung des Goldes (gestrichelte Linie) und des Nickels (geschlossene Linie) eines geschliffenen Katalysatorpartikel aus Vergleichsbeispiel 4 abgebildet. Man erkennt, die höhere Konzentration beider Metalle in den jeweiligen Außenbereichen der Partikel. Der steile Kurvenanstieg an den äußersten Rändern ist durch eine nicht glatte Oberfläche des Partikels zu erklären.

## Patentansprüche

1. Katalysatorpartikel für die oxidative Veresterung eines Aldehyds zu einem Carbonsäureester, **dadurch gekennzeichnet, dass** das Partikel die Elemente Sauerstoff, Silizium, Aluminium, ein basisches Element, Gold und mindestens ein Element ausgewählt aus Eisen, Zink und Kobalt enthält, und dass die maximale Gold- bzw. die maximale Eisen-, Zink- und/oder Kobaltkonzentration des Katalysatorpartikels in einem Außenbereich, der maximal 60 % des geometrischen Äquivalenzdurchmessers ausmacht, mindestens 1,5 mal höher ist als die Gold- bzw. Eisen-, Zink oder Kobaltkonzentration im mittleren Bereich, der den verbliebenen Bereich des geometrischen Äquivalenzdurchmessers ausmacht.

2. Katalysatorpartikel gemäß Anspruch 1, **dadurch gekennzeichnet, dass** das Partikel die Elemente Sauerstoff, Silizium, Aluminium, ein basisches Element, Gold und Kobalt enthält.

3. Katalysatorpartikel gemäß Anspruch 1 oder 2, **dadurch gekennzeichnet, dass** es sich bei dem basischen Elementen um ein Alkalimetall, ein Erdalkalimetall, ein Seltenerdemetall oder um Mischungen aus diesen Metallen handelt.

4. Katalysatorpartikel gemäß mindestens einem der Ansprüche 1 bis 3, **dadurch gekennzeichnet, dass** das Katalysatorpartikel bezogen auf die Gesamtmolmenge von Gold, Silizium, Aluminium und basischen Elementen, sowie Eisen-, Zink- und/oder Kobalt 0,03 bis 3 mol% Gold, 40 bis 90 mol% Silizium, 3 bis 40 mol% Aluminium, 2 bis 40 mol% des basischen Elements und 0,1 bis 20 mol% Eisen-, Zink- und/oder Kobalt enthält, wobei das Molverhältnis von Eisen-, Zink- und/oder Kobalt zu Gold zwischen 0,1 und 20 beträgt und alle dieser Elemente außer dem Gold als Oxide vorliegen.

5. Katalysatorpartikel gemäß mindestens einem der Ansprüche 1 bis 4, **dadurch gekennzeichnet, dass** das Katalysatorpartikel einen mittleren geometrischen Äquivalenzdurchmesser zwischen 10 und 250 µm aufweist, und dass die Dicke des Außenbereichs zwischen 2 und 100 µm liegt.

6. Katalysatorpartikel gemäß mindestens einem der Ansprüche 1 bis 5, **dadurch gekennzeichnet, dass** das Gold und/oder Gold- und Eisen-, Zink- und/oder Kobaltoxid-haltige Partikel mit einem mittleren Durchmesser zwischen 2 und 10 nm im Außenbereich des Katalysatorpartikels vorliegen.

7. Katalysatorpartikel gemäß mindestens einem der Ansprüche 1 bis 6, **dadurch gekennzeichnet, dass** sie porös sind, eine spezifische Oberfläche zwischen 100 und 300 m²/g aufweisen, und dass deren durchschnittlicher Porendurchmesser 1 bis 50 nm beträgt.

8. Katalysatorpartikel gemäß Anspruch 7, **dadurch gekennzeichnet, dass** die spezifische Oberfläche des Katalysatorpartikels zwischen 150 und 250 m²/g liegt, und dass der durchschnittliche Porendurchmesser 2 bis 20 nm beträgt.

9. Verfahren zur Herstellung eines Katalysatorpartikels gemäß mindestens einem der Ansprüche 1 bis 8, aufweisend die folgenden Schritte:
1) Auftragung mindestens einer Eisen-, Zink- und/oder Kobaltverbindung auf ein Partikel, bestehend aus den Oxiden des Siliziums, des Aluminiums und optional eines oder mehrerer Alkali-, Erdalkali- oder Seltenerdemetalle,
2) optionale partielle oder vollständige Oxidation des Materials aus 1) und optionale Trocknung/Kalzinierung,
3) Auftragung mindestens einer Goldverbindung auf das Material aus Schritt 2) und
4) Trocknung und/oder Kalzinierung des Materials aus Schritt 3).

10. Verfahren gemäß Anspruch 9, **dadurch gekennzeichnet, dass** in Verfahrensschritt 1) eine Kobaltverbindung aufgetragen wird.

11. Verfahren gemäß Anspruch 9 oder 10, **dadurch gekennzeichnet, dass** es sich bei Verfahrensschritt 2 um das Erhitzen in Gegenwart von Sauerstoff oder um die Zugabe eines Oxidationsmittels, insbesondere von H₂O₂, in eine wässrige Suspension des Partikels aus Verfahrensschritt 1 handelt.

12. Verfahren gemäß Anspruch 11, **dadurch gekennzeichnet, dass** es sich bei Verfahrensschritt 2 um eine Kalzinierung in Gegenwart von Sauerstoff handelt.

13. Verfahren gemäß mindestens einem der Ansprüche 9 bis 12, **dadurch gekennzeichnet, dass** das Partikel enthaltend die Oxide des Siliziums, des Aluminiums und optional eines oder mehrerer Alkali-, Erdalkali- oder Seltenerdemetalle innerhalb der Verfahrensschritte 1 bis 3 mindestens eine thermische Behandlung mit Wasser durchläuft, wobei die Wassertemperatur zwischen 50 und 100 °C beträgt.

14. Verfahren gemäß mindestens einem der Ansprüche 9 bis 13, **dadurch gekennzeichnet, dass** die Verfahrensschritte 1, 3 und optional 2 in einem wässrigen Medium durchgeführt werden, und dass die Verfahrensschritte 1 und 3 unter Verwendung einer wasserlöslichen Kobalt- bzw. wasserlöslichen Goldverbindung erfolgen.

15. Verfahren gemäß Anspruch 13, **dadurch gekennzeichnet, dass** die Wassertemperatur zwischen 70 und 95 °C liegt.

16. Verfahren gemäß mindestens einem der Ansprüche 9 bis 15, **dadurch gekennzeichnet, dass** in Verfahrensschritt 3 zu einer wässrigen Suspension der Partikel aus Verfahrensschritt 2 zuerst eine basische Lösung und anschließend eine Lösung, enthaltend Goldsäure, mit einem pH-Wert zwischen 0,5 und 5 gegeben werden.

17. Verfahren gemäß mindestens einem der Ansprüche 9 bis 15, **dadurch gekennzeichnet, dass** in Verfahrensschritt 3 zu einer wässrigen Suspension der Partikel aus Verfahrensschritt 2 eine Lösung, die durch die teilweise oder vollständige Neutralisation einer Goldsäure-Lösung, aufweisend einen pH-Wert zwischen 0,5 und 5, mit einer Base, bevorzugt mit einer NaOH-Lösung, erhalten wurde, gegeben wird.

18. Verwendung eines Katalysatorpartikels gemäß mindestens einem der Ansprüche 1 bis 8 zur oxidativen Veresterung eines Aldehyds in Gegenwart von Sauerstoff und einem Alkohol zu einem Carbonsäurester.

19. Verwendung eines Katalysatorpartikels gemäß Anspruch 18 zur oxidativen Veresterung von Methacrolein in Gegenwart von Sauerstoff und einem Alkohol zu einem Alkylmethacrylat.
